(19) **Europäisches Patentamt**

**European Patent Office**

**Office européen des brevets**

(11) **EP 1 674 077 A1**

(12) **EUROPEAN PATENT APPLICATION**

(43) Date of publication:
**28.06.2006 Bulletin 2006/26**

(51) Int Cl.:
***A61K 9/00*** *(2006.01)*

(21) Application number: **04106995.6**

(22) Date of filing: **27.12.2004**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Université de Liège**
**4020 Liège (BE)**

(72) Inventors:
 • **Herman, Ludivine,**
  **Université de Liège, Anatomie et**
  **4000 Liège (BE)**
 • **Hubert, Pascale, Dr.,**
  **Uni. de Liège, anatomie et**
  **4000 Liège (BE)**
 • **Delvenne, Philippe, Dr.,**
  **Uni. de Liège, anatomie**
  **4000 Liege (BE)**

 • **Boniver, Jacques, Prof.,**
  **Uni. de Liège, anatomie**
  **4000 Liège (BE)**
 • **Evrard, Brigitte, Prof.,**
  **Uni. de Liège, pharmacie**
  **4000 Liège (BE)**
 • **Delattre, Luc, Prof.,**
  **Uni. de Liège, pharmacie**
  **4000 Liège (BE)**
 • **Frankenne, Francis, Dr.,**
  **Uni. de Liège,**
  **4000 Liège (BE)**
 • **Noel, Agnes, Prof.,**
  **Uni. de Liège, obstetrique et**
  **4000 Liège (BE)**
 • **Foidart, Jean-Michel, Prof.,**
  **Uni. de Liège**
  **4000 Liège (BE)**

(54) **Mucoadhesive pharmaceutical compositions comprising chemoattractants**

(57) The invention relates to a mucoadhesive pharmaceutical composition comprising a polymer and a chemoattractant wherein the pH of the composition is greater than 6 which is useful in the treatment of a anogenital or oral disease, particularly an anogenital or oral disease cause by the human papillomavirus.

FIGURE 5

**Description**

The present invention relates to a composition for the treatment of a anogenital or oral disease, especially such a disease caused by a human papillomavirus.

Human Papillomaviruses (HPV) are common sexually transmitted pathogens inducing a spectrum of diseases ranging from benign genital warts to invasive carcinomas. Some types of HPV have been shown to be directly involved in the development of cervical cancer and its precursors (squamous intraepithelial lesions (SIL)) (Bosch et al, 1995). Squamous intraepithelial lesions refer to a group of premalignant changes in the epithelium of the uterine cervix that precede the onset of invasive cancer.

Additional environmental and/or host factors are probably involved in malignant progression as suggested by the small number of infected individuals developing cervical cancer and the relatively long latency period before cancer emergence. The role of the intrinsic immunity in controlling HPV infection and the subsequent development of SIL is shown indirectly by the increased frequency of HPV-associated lesions in patients with depressed cell-mediated immunity (Petry et al, 1994 ; Ellerbrock et al, 2000). The nature of an effective immune response to HPV infections is not well understood, although cell-mediated immunity is thought to be more important than humoral immunity (Thivolet et al, 1982; Wu et al, 1994). Several studies have described a localized immune dysfunction accompanying cervical HPV infections and associated cervical lesions.

A way of improving the treatment of such conditions has been sought.

According to the invention there is provided a mucoadhesive pharmaceutical composition comprising a polymer and a chemoattractant wherein the pH of the composition is greater than 6.

It has surprisingly been found that a chemoattractant significantly enhanced the chemotaxis of an antigen presenting cell and that the inclusion of a chemoattractant in a mucoadhesive composition having a pH greater than 6 stimulated the infiltration of an antigen presenting cell into a HPV-transformed epithelium. Therefore, the composition according to the invention will be useful in the treatment of a anogenital or oral disease, particularly an anogenital or oral disease caused by the human papillomavirus. A further useful feature of the invention is that the chemoattractant did not interfere with the antigen presenting ability of the antigen presenting cell.

A chemoattractant is generally understood to be a pharmacological agent which modulates the recruitment of cells such as a dendritic and/or Langerhans cell. The chemoattractant used in the invention is preferably selected from the group consisting of MIP3$\alpha$ (macrophage inflamatory proteine 3$\alpha$), H$\beta$D2 (human $\beta$ defensin 2), MCP-1 (monocyte chemotactic protein-1) and molgramostim. Preferably two or more chemoattractants are used, e.g. MIP3$\alpha$ and H$\beta$D2.

The composition according to the invention preferably contains an auxiliary chemoattractant which is selected from the group consisting of granulocyte macrophage - colony recruiting factor (GM-CSF) and HNP2 (human neutrophil defensin 2). A preferred combination of chemoattractant and auxiliary chemoattractant for use in the composition according to the invention is MCP-1 and HNP2.

The composition according to the invention is preferably in the form of a continuous structure, preferably having a solid-like property. The composition is optionally either clear or opaque. In the simplest composition according to the invention, the polymer is a natural gum (e.g. xanthane), a semi-synthetic material (e.g. methylcellulose, carboxymethylcellulose, hydroxyethyl-cellulose hydroxypropylmethyl-cellulose or hydroxypropyl-cellulose) or a synthetic material (e.g. a carbomer, polycarbophil and/or a carboxyvinyl polymer).

The polymer is preferably an acrylic acid containing polymer, more preferably it is a polycarbophil. The composition according to the invention is preferably in the form of a hydrogel.

The pH of the composition is preferably from 6 to 8. More preferably it is about 6.9.

The composition according to the invention optionally further comprises a preservative, buffer (especially an isotonic buffer) and/or stabiliser such as methylparahydroxybenzoate, parabens, EDTA, potassium sorbate and/or propylparahydroxybenzoate. A suitable carrier, excipient and/or other agent may be included in the composition according to the invention, e.g. to provide improved transfer and/or delivery.

According to the invention there is also provided a composition according to the invention for use in the treatment of a squamous mucosa, preferably in the treatment of an anogenital or oral disease, especially human papilloma virus.

By anogenital disease is meant vulvar, vaginal, cervical and/or penile and/or anorectal disease.

According to the invention there is further provided use of a composition according to the invention in the manufacture of a medicament for use in the treatment of a squamous mucosa, preferably in the treatment of an anogenital or oral disease, especially human papillomavirus.

According to the invention there is also provided a method of treating an anogenital and/or oral disease which method comprises administering a therapeutically effective amount of a composition according to the invention to a patient in need of such treatment. Typically, treatment with direct administration is made daily, weekly or monthly for a period of time sufficient to reduce, prevent or ameliorate one or more symptoms.

For a typical composition having a volume of about 100ml, the amount of chemoattractant used in the composition is from 10ng to 500$\mu$g, preferably from 100ng to 100$\mu$g, depending on the chemoattractant used. A person of skill in the art will be able to determine a suitable amount.

The invention is illustrated with reference to the Figures of the accompanying drawings in which:

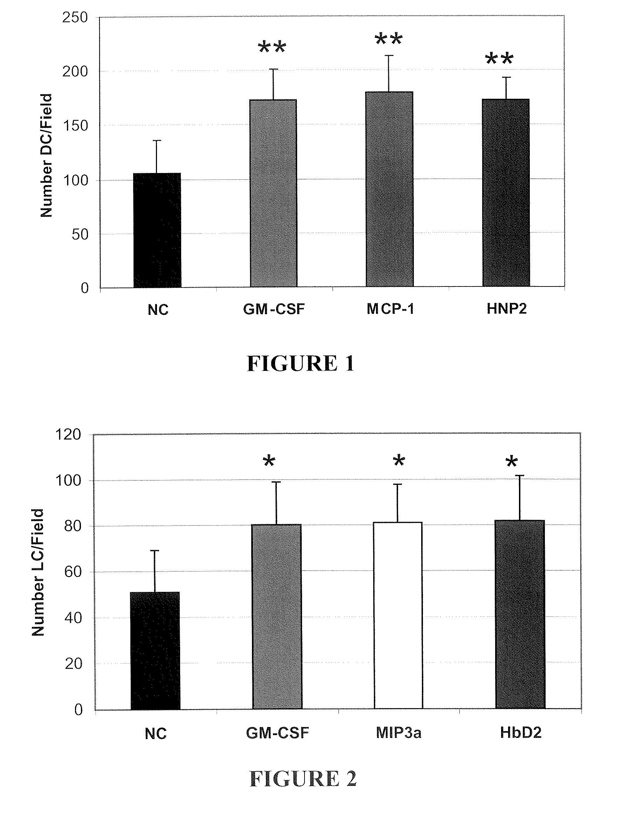

Figure 1 is a graph showing chemotactic activity of different molecules on DC wherein NC indicates non-conditioned medium; the results are the mean $\pm$ SD of three experiments and asterisks indicate statistically significant differences: **P<0.01;

Figure 2 is a graph showing chemotactic activity of different molecules on LC wherein NC indicates non-conditioned medium; results are the mean $\pm$ SD of seven experiments and asterisks indicate statistically significant differences: *P<0.05;

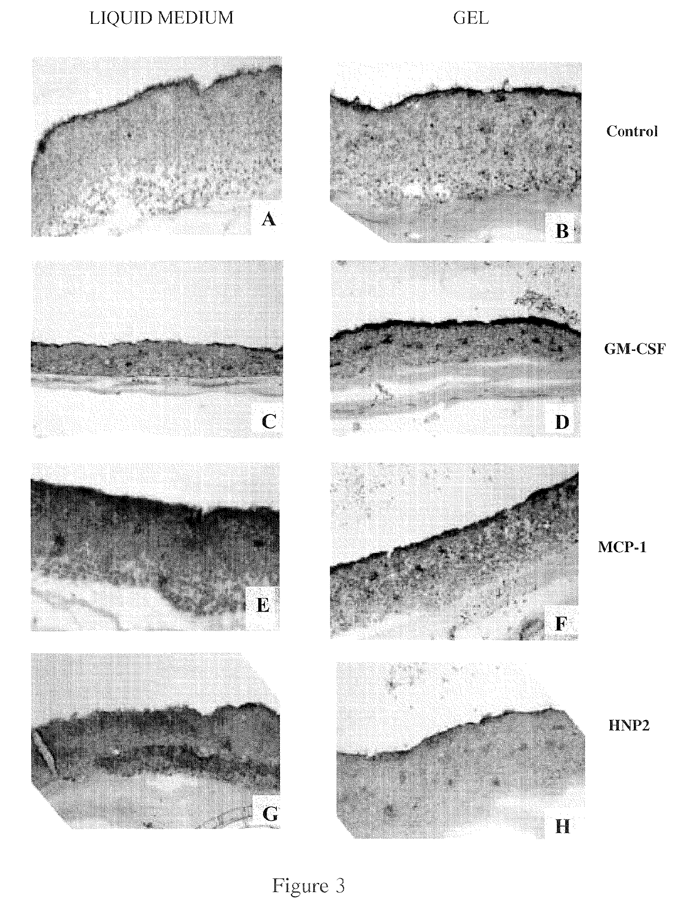

Figure 3 shows the correlation between the penetration of DC into organotypic cultures and the addition of exogenous GM-CSF(C,D); MCP-1(E,F); and HNP2(G,H), shown by CD1a$^+$ immunolabeled sections of organotypic culture of HPV$^+$ keratinocytes wherein photographs A and B show DC infiltration in the absence of chemotactic molecules and C-H show DC recruitment in the presence of chemotactic molecules in liquid culture medium (C, E, G) or included in the polycarbophil gel (D, F, H);

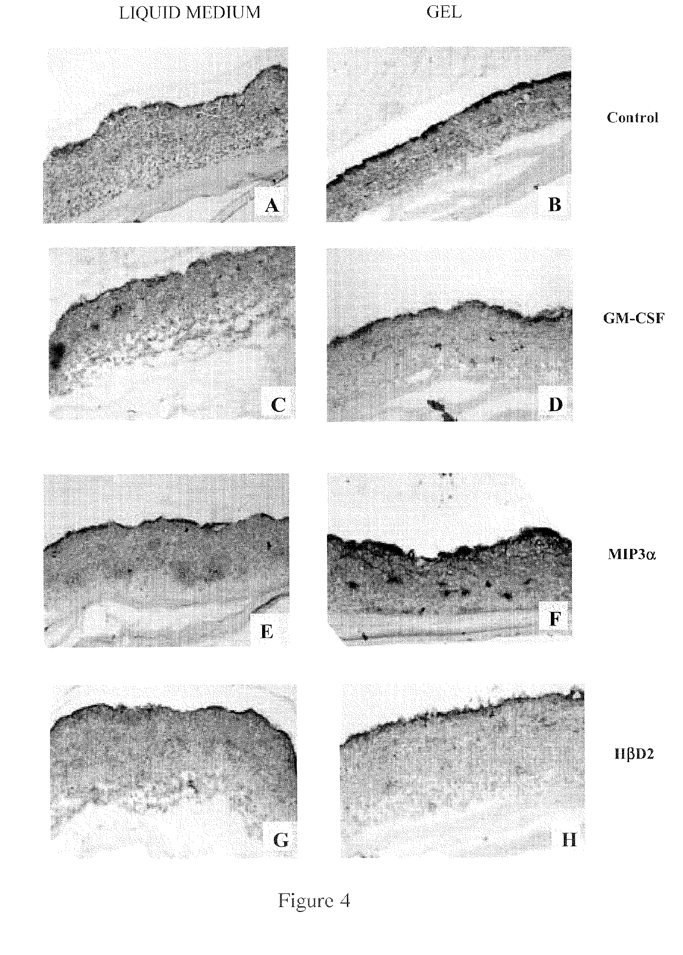

Figure 4 shows the correlation between the penetration of LC into organotypic cultures and the addition of exogenous GM-CSF (C,D); MIP3$\alpha$ (E,F); and H$\beta$D2 (G,H), shown by CD1a$^+$ immunolabeled sections of organotypic culture of HPV$^+$ keratinocytes wherein photographs A and B show DC infiltration in the absence of chemotactic molecules and C-H show DC recruitment in the presence of chemotactic molecules in liquid culture medium (C, E, G) or included in the polycarbophil gel (D, F, H);

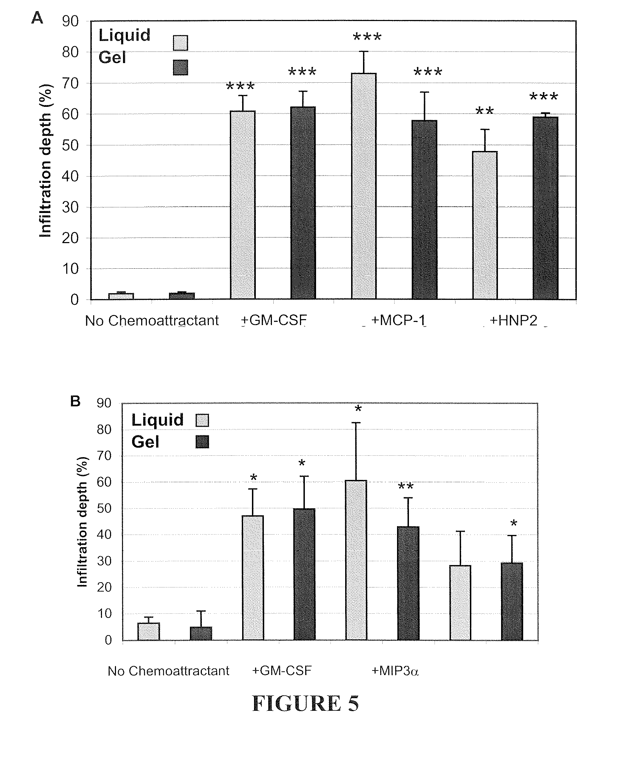

Figure 5 shows quantitative evaluation of DC and LC infiltration into organotypic cultures of HPV-transformed keratinocytes wherein the penetration of DC (A) is tested in the absence or in the presence of GM-CSF (used as control), MCP-1 and HNP2 included or not in polycarbophil gel; and the infiltration of LC (B) is evaluated in the absence or in the presence of GM-CSF, MIP3$\alpha$ and H$\beta$D2 included or not in the polycarbophil gel. DC and LC were detected by immunolabeling with anti-CD1a antibody. Results are expressed as infiltration depth in percentage of epithelial sheet thickness (n=4 for each culture condition). Asterisks indicate statistically significant differences: *P<0.05; **P<0.01; ***P<0.001;

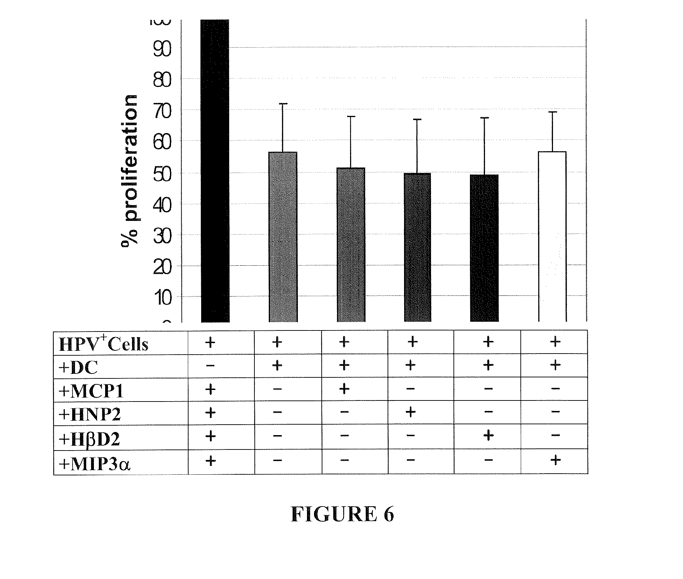

Figure 6 shows that DC-mediated cytostatic activity is maintained in the presence of chemokines and defensins. HPV$^+$ keratinocytes were cultured in 96-well plates at $5 \times 10^3$ cells/well. These cells were cultured with DC added at $4 \times 10^4$ cell/well. After an incubation of 48h in the presence or in the absence of chemotactic molecules, cell proliferation was measured by 3H-TdR incorporation. The results are expressed as percentages of HPV$^+$ keratinocyte proliferation in the coculture compared to keratinocytes cultured alone, in the presence or in the absence of chemokines or defensins. The results are presented as means $\pm$ SD of 4 experiments using DC from different donors; and

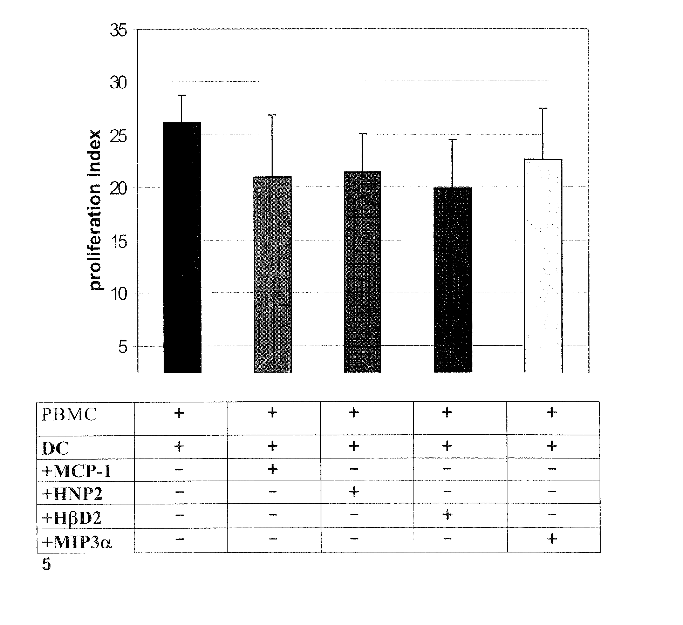

Figure 7 shows that Chemokines and defensins did not alter the ability of DC to stimulate a potent mixed lymphocyte reaction. $5 \times 10^4$ DC irradiated with 2500 rads were cultured with $1 \times 10^5$ PBMC in the presence of chemokines or defensins, during 7 days. The cell proliferation was measured by 3H-TdR incorporation. Proliferation index was obtained by comparing the cell proliferation in the mixed DC-PBMC cultures added or not with chemokines/defensins, with PBMC cultured alone with or without chemokines/defensins. These results are presented as means $\pm$ SD of 3 experiments using DC from different donors.

The invention will now be illustrated with reference to the following Examples which are not intended to limit the scope of the invention defined herein.

EXAMPLE 1

A gel formulation according to the invention was prepared in the usual manner from a therapeutically effective amount of MCP-1, MIP3$\alpha$ or H$\beta$D2, 3 wt.% hydroxyethylcellulose (TYLOSE® H4000), 0.3 wt.% of potassium sorbate, 0.3 wt.% of $NaH_2PO_4$ and purified water q.s. to 100 wt.%.

EXAMPLE 2

A gel formulation according to the invention suitable for oral or anal delivery was prepared in the usual manner from a therapeutically effective amount of MCP-1, MIP3$\alpha$ or H$\beta$D2 with 7g of hydroxypropylcellulose (Klucel GF) and an isotonic buffer to 100g. The isotonic buffer contains 1.36 g of $KH_2PO_4$, 200 ml of purified water and 4N NaOH to give a pH of 6.9.

EXAMPLE 3

A gel formulation according to the invention suitable for oral or anal delivery was prepared in the usual manner from a therapeutically effective amount of MCP-1, MIP3α or HβD2 with 20 g of polyethylene polyoxypropylene block polymer (Lutrol F127) and an isotonic buffer as defined in Example 2 to 100g.

EXAMPLE 4

A gel formulation according to the invention suitable for oral or anal delivery was prepared in the usual manner from a therapeutically effective amount of MCP-1, MIP3α or HβD2 with 1.5g of polyacrylic acid (Carbopol 974P), 0.5g of EDTA, trometamol to a pH of 6.9 and purified water to 100g.

EXAMPLE 5

A gel formulation according to the invention suitable for oral or anal delivery was prepared in the usual manner from a therapeutically effective amount of MCP-1, MIP3α or HβD2 with:

| | |
|---|---|
| Hydroxypropylcellulose (Klucel GF) | 7g |
| Parabens | q.s. |
| Isotonic buffer (as in Example 2) | to 100g |

EXAMPLE 6

A gel formulation according to the invention suitable for oral or anal delivery was prepared in the usual manner from a therapeutically effective amount of MCP-1, MIP3α or HβD2 with:

| | |
|---|---|
| Polyethylene polyoxypropylene block polymer (Lutrol F127) | 20 g |
| Parabens | q.s. |
| Isotonic buffer (as in Example 2) | to 100g |

EXAMPLE 7

A gel formulation according to the invention suitable for oral or anal delivery was prepared in the usual manner from a therapeutically effective amount of MCP-1, MIP3α or HβD2 with:

| | |
|---|---|
| Polyacrylic acid (Carbopol 974P) | 1.5 g |
| EDTA | 0.5 |
| Parabens | q.s. |
| Purified water | to 100g |
| Trometamol | to pH 6.9 |

EXAMPLE 8

MATERIALS AND METHODS

Culture of cervical HPV- transformed keratinocyte cell lines

SiHa and CasKi are tumorigenic cervical carcinoma-derived keratinocyte cell lines (Friedl et al, 1970; Pater et al, 1985; Auersperg et al, 1962). SiHa cell line contains one copy and CasKi contains approximately 600 copies of integrated HPV-16 DNA. These HPV-transformed keratinocyte cell lines were cultured in growth medium composed of 1/3 mixture of HAM F12 (GIBCO BRL, Nord Island, NY, USA)/Dulbecco's modified Eagle's medium (GIBCO BRL), supplemented with 0.4 μg/ml hydrocortisone (Sigma Chemical Co., St Louis, MO), 2 ng/ml epidermal growth factor (Sigma), 10 % fetal calf serum (Life Sciences International, Zelik, Belgium), 2 mmol/L L-glutamine (GIBCO BRL), 10 mmol/L Hepes (GIBCO BRL), 1 $\mu$g/ml fungizone (GIBCO BRL), 1 mmol/L sodium pyruvate (GIBCO BRL), 3000 U/ml penicillin-streptomycin (GIBCO BRL), $10^{-10}$ mol/L cholera toxin (Sigma), 5 $\mu$g/ml insulin (Sigma), 20 $\mu$g/ml adenine (Sigma), 5 $\mu$g/ml human transferrin (Sigma), and $15.10^{-4}$ $\mu$g/ml 3,3',5-triiodo-L-thyronine (Sigma).

Dendritic cell cultures

Dendritic cells (DC) were generated by culturing adherent fraction of human PBMC as previously described (Hubert et al, 1998; Sallusto and Lanzavecchia, 1994). Briefly, Peripheral Blood Mononuclear Cells (PBMC) were isolated from leukocyte-enriched buffy-coats by centrifugation on Ficoll-Hypaque. After washings at low centrifugation speed to discard a maximum of platelets, PBMC were plated in six-well dishes (Nunclon Multidishes, NUNC, Denmark) at a density of $10\times10^6$ PBMC per well in 3 ml of medium without FCS. After 18h at 37°C, non-adherent cells were discarded by two gentle washes and the plastic adherent fraction was cultured with 800 U/ml human recombinant GM-CSF (Amoytop Biotech, Xiamen, China) and 40 U/ml IL4 (ImmunoTools, Friesoythe, Germany) in 3 ml of RPMI 10% FCS-50$\mu$M Mercaptoethanol. Cultures were fed every 3 days with fresh medium containing cytokines and harvested with PBS-EDTA 1mM at day 7.

Langerhans cell cultures

Cord blood mononuclear cells (CB MNC) were recovered after discontinuous density gradient centrifugation using Lymphoprep™ (Nycomed Pharma As, Oslo, Norway) within 24 hours after collection. CD34+ cells were isolated from CB MNC using the MACS Direct CD34 Progenitor Cell Isolation Kit (Miltenyi Biotec GmBH, Bergisch Gladbach, Germany) and MiniMACS separation columns (Miltenyi Biotec) according to the manufacturer's protocol. Fifteen $10^4$ CD34+ cells were seeded in T25 flasks (Sarstedt, Inc, Newton, NC) in 10 ml of RPMI 1640 medium supplemented with 10% FCS, antibiotics and 50 $\mu$M mercaptoethanol (all from GIBCO-BRL). Cultures were supplemented with previously optimized concentrations of the following human molecules: SCF (20 ng/ml, specific activity (SA) > 5 $10^5$ U/mg), TPO (10 U/ml, SA >1 $10^6$ U/mg), F1T3-L (25 ng/ml, SA >2 $10^5$ U/mg), GM-CSF (200 U/ml, SA: 11,1 $10^6$ U/mg), TNF$\alpha$ (50 U/ml, SA >2 $10^7$ U/mg), IL4 (100 U/ml, SA >2 $10^6$ U/ml) and TGF-$\beta$1 (5 ng/ml or 12.5 ng/ml, SA >2 $10^8$ U/mg). All these agents were purchased from PeproTech (Rockey Hill, NJ), except for GM-CSF and IL4 which were obtained from Amoytop (Amoytop Biotech, Xiamen, China) and Biosource (Nivelles, Belgium), respectively. The cells were cultured at 37°C in a humidified atmosphere and in the presence of 5% $CO_2$. At day 7, the cellular density was adjusted to $2.10^4$ per $cm^2$ and the cells were fed, at days 7 and 14, with GM-CSF, IL4, TNF$\alpha$ and TGF-$\beta$1 at the same concentrations as those used at the start of the culture, except for TGF-$\beta$1 at day 14 which was 12.5 ng/ml for all the cultures. At day 18, cells were collected from cultures by vigorous pipetting to prepare single cell populations (Hubert et al, in press).

Chemotaxis assay

Cell migration was evaluated using a chemotaxis microchamber technique (48-well Boyden microchamber; Neuroprobe, Cabin John, MD,USA). The lower wells of the chemotaxis chamber was filled with 27 of non conditioned medium, human fibroblasts derived cell-conditioned medium or the differents chemokines studied. Nonconditioned medium was used as control for random migration. Each condition was repeated six times. After phenotypic characterisation, DC were harvested and 55$\mu$l of DC suspension ($2x10^6$ cells/ml) were applied to the upper wells of the chamber, with a polyvinylpyrollidone-free polycarbonate membrane 8-$\mu$m pore filter (Poretics Corp., Livermore, CA) separating the lower wells. These membranes were coated by incubation with 100$\mu$g/ml gelatin in 0.1% acetic acid solution. The chamber was incubated for 5h at 37°C in a 5% $CO_2$/95% air atmosphere. The cells having migrated to the underside of the filter were fixed and stained with Diff Quick Stain set (Baxter Diagnostics AG, Düdingen, Switzerland). The upper side of the filter was scraped to remove residual non migrating cells. One random field was counted per well using an eyepiece with a calibrated grid to evaluate the number of fully migrated cells.
The same tests were realized for LC using standard 5-$\mu$m pore polyvinylpyrollidone-free polycarbonate filters to separate the upper and the lower wells.

Preparation of the hydrogel

Polycarbophil (Noveon AA1) was supplied from Noveon (Brussels, Belgium). The polycarbophil gel (1.5% w/w) was prepared by dispersing the Noveon AA1 in keratinocyte growth medium. The mixture was stirred until thickening occurred and then neutralized by dropwise addition of 40% (w/w) trometamol, until a transparent gel appeared. The quantity of trometamol was adjusted to achieve pH 7.

Organotypic Cultures

Organotypic cultures of HPV-transformed keratinocyte cell lines were prepared by procedures slightly modified from those described previously (Delvenne et al, 1995; Merrick et al, 1992). For the preparation of dermal equivalents, a collagen matrix solution was made with 32 mg of collagen (cellagen solution AC-5, type I, ICN, Biomedical, Asse-Relegen, Belgium) mixed on ice with 1.6 ml of 0.1% acetic acid, 1ml of chilled 10-fold concentrated Hanks' buffer supplemented with phenol red and 1N NaOH to give a pH of 7.2. One milliliter of FCS containing 5x10$^5$ normal human fibroblasts was then added. One milliliter of collagen/fibroblast solution was poured into 24-well plates (Nunclon Multidishes, Nunc, Roskilde, Denmark) and allowed to solidify at 37°C for 1 hour. The final concentrations of collagen and fibroblasts were 3.2 mg/ml and 5x10$^4$ cells/ml, respectively. After gel equilibration with 1ml of growth medium overnight at 37°C, 30.10$^4$ HPV-transformed keratinocytes resuspended in 50µl of growth medium were seeded on top of the gels and maintained submerged for 48 hours. The collagen rafts were raised in 25-mm tissue culture insert (8-µm pore size; Nunc) and placed onto stainless-steel grids at the interface between air and liquid culture medium. Epithelial cells were then allowed to stratify over 20 days. After stratification of keratinocytes, DC were seeded on top of the in vitro-formed epithelium at a concentration of 4.10$^5$ cells/50µl of keratinocyte growth medium. The liquid culture medium or the polycarbophil gel was supplemented or not with the different chemokines/defensins at the following concentrations: 500 ng/ml of Monocyte Chemotactic Protein 1 (MCP-1, PeproTech, Rocky Hill, NJ), 750 ng/ml of Human Neutrophil Peptide-2 (HNP2, Sigma-Aldrich, St Louis, MO), 750 ng/ml of Human β Defensin-2 (HβD2, PeproTech, Rocky Hill, NJ) or 500 ng/ml of Macrophage Inflammatory Protein 3α (MIP3α, PeproTech, Rocky Hill, NJ). After 48 hours at 37°C, the collagen rafts were harvested. The cultures were then embedded in OCT compound (Tissue Tek, Sakura, The Netherlands) at -70°C and sectioned with a cryostat microtome for the immunohistochemical analysis.

Immunohistochemistry

The density of DC migrating into the epithelial layer was assessed by the avidin-biotin-peroxidase technique (Vectastain ABC kit, Vector Laboratories, Burlingame, CA) with an anti-CD1a monoclonal antibody (clone NA1/34 from Dako, Glostrup, Denmark). Eight-micron frozen sections were fixed in cold acetone for 3 minutes, and endogenous peroxidases were blocked with 0.1% $H_2O_2$ for 30 minutes. Sections were then incubated sequentially with anti-CD1a antibody (at a 1/40 dilution in PBS containing 2% bovine serum albumin (BSA)) for 1 hour, with a biotinylated mouse anti-Ig antibody for 30 minutes, and with streptavidin/horseradish peroxidase/avidin/biotin complex for another 30 minutes. Positive cells were visualized by a 3,3'-diaminobenzidine substrate (DAB). The sections were counterstained with hematoxylin.
Assessment of CD1a+ Cell Infiltration in Organotypic Cultures.
The DC/LC infiltration in the organotypic cultures was evaluated by measuring the distance between the top of the epithelium and individual DC with a computerized system of image analysis (CAS, Becton Dikinson, Erembodegem, Belgium). The ratio between the infiltration depth and the thickness of the culture was then calculated. The percentage ratio was 0% for DC/LC staying on the top of the culture and 100% for DC/LC reaching the bottom of the epithelium.

Keratinocyte growth inhibition assay

HPV-transformed keratinocyte cell lines (5x10$^3$ cells/well) with or without DC (40000 DC/well) were cultured in 96-well plates (Nunclon Surface, NUNC) with or without chemokines/defensins. Proliferation was measured after 48h at 37°C, following an 18 h incubation with 0.4µCi/well of [$^3$H]thymidine (6,7Ci/mmol, Moravek Biochemicals, Brea, CA). [$^3$H]thymidine can incorporate DNA during the S phase of dividing cell cycle. DNA was harvested by an automated sample harvester (Packar, Canberra, Tilburg, The Netherlands) and thymidine incorporation was analysed by using a liquid scintillation counter (Top Count, Packard, Canberra). The data are presented as the percentage of proliferation calculated by using the following formula:

$$\% \text{ proliferation} = (\text{test cpm/control cpm}) \times 100$$

where test cpm is [$^3$H]thymidine incoporation by keratinocytes cultured with DC in the presence or not of chemokines/defensins and control cpm is [$^3$H]thymidine incorporation by keratinocytes without DC but with or without chemokines/defensins.

Mixed lymphocyte reaction assay

Stimulator populations (dendritic cells) were harvested and irradiated with 2500 rads. After a wash and a centrifugation, the cells were adjusted to 50000 DC/100μl in RPMI-1640 medium containing 5% human pooled AB serum. Stimulator cells (50000 DC/well) were then added to roundbottomed 96-well plates (Nunclon) containing $1 \times 10^5$ allogeneic PBMC per well and the differents chemokines/defensins. The tests were performed in quadruplate. A proliferative response was measured after 7 days of culture by adding 0.4 μCi [³H]thymidine (6,7Ci/mmol, Moravek Biochemicals, Brea, CA) to each well. DNA was harvested 18h later by an automated sample harvester (Packar, Canberra, Tilburg, The Netherlands) and counted in a liquid scintillation counter (Top Count, Packard, Canberra).

Statistical analysis

Statistical analysis was performed by using the Unpaired Student t test (Instat Mac 2.01 software; Graph-Pad Software, San Diego, CA). Differences were considered statistically significant when $P < 0.05$.

RESULTS

HNP2 and MCP-1 induce the chemotaxis of DC

To identify molecules that could influence the migration of DC, we conducted a migration assay using a Boyden microchemotaxis chamber. DC generated for this study were judged to be 90% pure based on several criteria, including morphology, forward-scatter and side-scatter values by flow cytometry, and surface phenotype (data not shown).
As shown in Figure 1, the addition of GM-CSF (1ng/ml) known to be chemoattractant for DC (Hubert et al, 1999) significantly enhanced the chemotaxis of DC, compared with the nonconditioned medium used to assess the chemokinesis. Interestingly, MCP-1 and HNP2 increased the migration of DC at respectively 500 ng/ml and 750 ng/ml which were previously determined in a preliminary work to be the optimal concentrations. Besides, the chemotactic activity of MCP-1 and HNP2 was similar to the level of chemotaxis induced by GM-CSF.

HβD2 and MIP 3α induce the chemotaxis of LC

In order to determine whether LC could be attracted by chemotactic molecules, Boyden chamber assay was performed with in vitro generated LC. LC were generated from CD34⁺ cord blood progenitors cultivated in the presence of hematopoïetic growth factors (TPO, SCF, Flt3L), cytokines (GM-CSF, TNFα, IL4) and TGF-β1(Hubert et al, in press). They exhibited morphological, immunohistochemical (CD1a⁺, CD207⁺, E-cadherin⁺, CLA⁺ and CCR6⁺) and ultrastructural features (Birbeck granules) of LC as verified by FACS analysis and electron microscopy (data not shown).
As observed for DC, GM-CSF (10ng/ml) induced a significantly higher LC migration than the nonconditioned medium. The mobility of LC in the presence of MIP3α 500 ng/ml) or HβD2 (750 ng/ml) was identical to that observed with GM-CSF (Figure 2).
Chemokines and defensins, included in a polycarbophil gel stimulate the infiltration of DC/LC into organotypic cultures of HPV-transformed keratinocytes.
It was investigated whether the addition of MIP3α and HβD-2 or MCP-1 and HNP2 could modulate the ability of LC and DC respectively to infiltrate an *in vitro*-formed (pre)neoplastic epithelium, reminiscent of a cervical high-grade lesion observed *in vivo*. After 20 days of culture, the HPV-transformed keratinocyte cell lines grown on a collagen gel at the air/liquid interface, produced an epithelial layer of up to 10-15 cells in thickness. These cells appeared disorganized and highly atypical throughout the full thickness of the epithelium as observed in high-grade lesion biopsies.
DC or LC were layered on the top of these cultures in the presence or in the absence of chemoattractant molecules. In order to evaluate the possibility to topically apply these molecules in an immunotherapeutic approach, chemokines and defensins were included in a polycarbophil gel. The ability of chemotactic molecules to influence the migration of DC/LC was determined by evaluating the density of CD1a⁺ cells in sections of organotypic cultures at 48 hours following chemoattractant addition. Figure 3 illustrates representative experiments showing CD1a-labeled DC in HPV-transformed organotypic cultures incubated or not with chemotactic molecules included or not in polycarbophil gel. Figure 4 represents similar experiments performed with LC.
In HPV-transformed keratinocyte organotypic cultures, LC/DC poorly infiltrated the epithelial layer in the absence of chemoattractant (Figure 3, A-B and figure 4, A-B). In contrast, addition of GM-CSF, the positive control, caused a significant increase in the density of LC/DC observed in the epithelial layer (Figure 3, C-D and Figure 4, C-D). The addition of chemokines and defensins in growth medium induced a recruitment of DC/LC similar to that observed with GM-CSF (Figure 3 C,E,G and Figure 4 C,E,G). Interestingly, this infiltration was also present when the chemoattractants were included in the polycarbophil gel (Figure 3 D, F, H and Figure 4 D, F, H).

Quantitative analysis of DC/LC infiltration was perfomed by evaluating the infiltration depth of all CD1a$^+$ cells throughout the full thickness of organotypic cultures (Figure 5).

When the medium of organotypic culture was supplemented with MCP1 or HNP2, the infiltration of DC increased compared to the basal infiltration without chemoattractant, and reached an infiltration level equivalent to that obtained with GM-CSF (Figure 5, A). The inclusion of chemoattractant molecules in the polycarbophil gel did not modify the DC infiltration compared with that observed in the presence of chemoattractants in the culture medium.

The infiltration of LC was also increased in the presence of MIP3$\alpha$ or H$\beta$D-2 (Figure 5, B). When the medium of organotypic cultures was supplemented with H$\beta$D-2, the migration of LC was slightly lower compared with that observed in the presence of GM-CSF. The addition of MIP3$\alpha$ induced a recruitment similar to that obtained with GM-CSF. The chemoattractants included in liquid culture medium or in polycarbophil gel recruited LC in a similar manner.

To determine the potential influence of chemokines/defensins on DC/LC differentiation, we performed a double-immunostaining (CD1a/CD14) of organotypic cultures. The phenotype of DC/LC infiltrating organotypic cultures in the presence of chemokines/defensins was not altered compared with that established before the migration (data not shown).

The cytostatic activity of DC against HPV+ keratinocytes is not modified in the presence of chemokines/defensins.

Since DC are able to inhibit the growth of HPV-transformed keratinocytes (Hubert et al, 2001) in vitro, we wanted to determine the influence of chemotactic molecules on this cytostatic activity. We performed a 48h growth-inhibition assay by coculturing HPV$^+$ keratinocytes with DC in the presence or not of different chemoattractants.

DC exhibited a significant growth inhibition effect on HPV$^+$ keratinocytes since DC reduced the proliferation of HPV$^+$ keratinocytes to 56% compared to cultures without DC. Neither defensins nor chemokines modified the cytostatic activity of DC against HPV$^+$ keratinocytes.

Chemokines and defensins do not affect the antigen presentation function of DC in a Mixed lymphocyte reaction (MLR)

The lymphocyte proliferation was not changed in the presence of chemokines or defensins only (data not shown).

As shown in Figure 7, DC in the presence or in the absence of chemotactic molecules did not differ significantly in their ability to stimulate the T lymphocyte proliferation. Consequently, the capacity of DC to present antigen to T lymphocytes was not affected.

REFERENCES

1. Albert ML, Sauter B, Bhardwaj N. Dendritic cell acquire antigen from apoptotic cells and induce class I restricted CTLs. Nature 1998, 392:86-9.

2. A1-Saleh W, Delvenne P, Anesse JE, Nikkels A, Pierard GE, and Boniver J. Inverse modulation of intraepithelial Langerhans cell and stromal macrophage /dendrocyte populations in human papillomavirus-associated squamous intraepithelial lesions of the cervix. Virchows Arch 1995, 427:41-48.

3. Al-Saleh W, Giannini SL, Jacobs N, Moutschen N, Doyen J, Boniver J, and Delvenne P. Correlation of T-helper secretory differentiation and types of antigen-presenting cells in squamous intraepithelial lesions of the uterine cervix. J Pathol 1998, 184:283-290.

4. Auersperg N, Hawryluk AF. Chromosome observations on three epithelial cell cultures derived from carcinomas of the uterine cervix. J Natl Cancer Inst 1962, 28:605-627.

5. Bell D, Chomarat P, Broyles D, et al. In breast carcinoma tissue, immature dendritic cells reside within the tumor, whereas mature dendritic cells are located in peritumoral areas. J Exp Med 1999, 190:1417-26.

6. Blanton RA, Perez-Reyes N, Merrick DT, McDougall JK. Epithelial cells immortalized by human papillomaviruses have premalignant characteristics in organotypic cultures. Am J Pathol 1991, 138:673-685.

7. Bosch FX, Manos MM, Munoz N, Sherman M, Jansen AM, Peto J.
Prevalence of human papillomavirus in cervical cancer : a worldwide perspective. International Biological Study on cervical cancer 5IBBCC) study Group. J Natl Cancer Inst 1995, 87:796-802.

8. Charbonnier AS, Kohrgruber N, Kriehuber E, et al. Macrophage inflammatory protein 3 alpha is involved in the constitute trafficking of epidermal Langerhans cells. J Exp Med 1999, 190:1755-68.

9. Delvenne P, Al-Saleh W, Gilles C, Thiry A, Boniver J. Inhibition of growth of normal and human papillomavirus-transformed keratinocytes in monolayer and organotypic cultures by interferon—$\gamma$ and tumor necrosis factor-$\alpha$. Am J Pathol 1995, 146:589-598.

10. Ellerbrock TV, Chiasson MA, Bush TJ, et al. Incidence of cervical squamous intraepithelial lesions in HIV-infected women. JAMA 2000, 283:1031-7.

11. Friedl F, Kimura I, Osato T, Ito Y. Studies on a new human cell line (SiHa) derived from carcinoma of uterus. I. Its establishment and morphology. Proc Soc Exp Biol Med 1970, 135:543-545.

12. Furukawa T, Watanabe S, Kodama T, Sato Y, Shimosat Y, and Suematsu K. T-zone histiocytes in adenocarcinoma of the lung in relation to postoperative prognosis. Cancer 1985, 56:2651-2656.

13. Giannini SL, Al-Saleh W, Piron H, Jacobs N, Doyen J, Boniver J, and Delvenne P. Cytokines expression in squamous intraepithelial lesions of the uterine cervix : implications for the generation of local immunosuppression. Clin Exp Immunol 1998, 113:183-189.

14. Giannini SL, Hubert P, Doyen J, Boniver J, and Delvenne P. Influence of the mucosal epithelium microenvironnement on Langerhans'cells : intraepithelial lesions of the cervix. Int J Cancer 2002, 10:654-659.

15. Granelli-Piperno A, Moser B, Pope M, Chen D, Wei Y, Isdell F, O'Doherty U, Paxton W, Koup R, Mosjov S. Efficient interaction of HIV-1 with purified dendritic cells via multiple chemokines receptors. J Exp Med 1996, 184: 2433-2438.

16. Hubert P, Greimers R, Franzen-Detrooz E, Doyen J, Delanaye P, Boniver J, Delvenne P. In vivo propagated dendritic cells from patients with human-papillomavirus-associated preneoplastic lesions of the uterine cervix : use of Flt3 ligand. Cancer Immunol Immunother 1998, 47:81-89.

17. Hubert P, van den Brûle F, Giannini SL, Franzen-detrooz E, Boniver J, and Delvenne P. Colonization of in vitro-formed cervical human papillomavirus-associated (pre) neoplastic lesions with dendritic cells : role of granulocyte/macrophage colony-stimulating factor. Am J Pathol 1999, 154:775-784.

18. Hubert P, Giannini SL, Vanderplasschen A, Franzen-detrooz E, Jacobs N, Boniver J, and Delvenne P. Dendritic cells induce the death of human papillomavirus transformed keratinocytes. FASEB J 2001, 15:2521-2523.

19. Hubert P, Evrard B, Maillard C, Franzen-detrooz E, Delattre L, Foidart JM, Noël A, Boniver J, and Delvenne P. A bioadhesive hydrogel of GM-CSF stimulates the migration of dendritic cells in models of HPV-associated (pre) neoplastic epithelium. Antimicrob Agents Chemother 2004, in press.

20. Hubert P, Bousarghin L, Greimers R, Franzen-detrooz E, Boniver J, and Delvenne P. Production of large numbers of Langerhans cells with intraepithelial migration ability in vitro. Exp Dermatol 2004, in press.

21. Hui W, Yoko NS, Toshikazy K, Mariko A, and Naofumi M. Potential involvement of monocyte chemoattractant protein (MCP)-1/CCL2 in IL-4-mediated tumor immunity through inducing dendritic cell migration into the draining lymph nodes. Int Immunopharmacol 2003, 3:627-642.

22. Jacobs N, Giannini SL, Doyen J, Baptista A, Moutshen M, Boniver J, and Delvenne P. Inverse modulation of IL10 and IL12 in the blood of women with preneoplastic lesions of the uterine cervix. Clin Exp Immunol 1998, 111: 219-224.

23. Jonuleit H, Knop J, Enk AH. Cytokines and their effects on maturation, differentiation and migration of dendritic cells. Arch Dermatol Res 1999, 289:1-8.

24. Kaplan G. Recent advances in cytokine therapy in leprosy. J Infect Dis 1993, 167:S18-S22.

25. Kleine-Lowinski K, Rheinwald JG, Fichorova RN, Anderson DJ, Basile J, Munger K, Daly CM, Rosl F, Rollins BJ. Selective suppression of monocyte chemoattractant protein-1 expression by human papillomavirus E6 and E7oncoproteins in human cervical epithelial and epidermal cells.Int J Cancer 2003, 107:407-15.

26. Knuth K, Amiji M, and Robinson J. Hydrogel delivery system for cervico-vaginal and oral applications: formulations and biological consideration Adv Drug Deliv Rev 1993, 11:137-167.

27. Macatonia SE, Gompels M, Pinchin AJ, et al. Antigen presentation by macrophages but not by dendritic cells in human immunodeficiency virus (HIV) infection. Immunology 1992, 75:576-581.

28. Merrick DT, Blanton RA, Gow AM, McDougall JK. Altered expression of proliferation and differentiation markers in human papillomavirus 16 and 18 immortalized epithelial cells grown in organotypic cultures. Am J Pathol 1992, 140:167-177.

29. Morelli AE, Sananes C,Di Paola G, Paredes A, Frainboim L. Relationship between types of human papillomavirus and Langerhans'cells in cervical condyloma and intraepithelial neoplasia. Am J Clin Pathol 1993, 99:200-207.

30. Müller CA, Markovic-Lipkovski J, Klatt T, Gamper J, Schwarz G, Beck H, Deeg M, Kalbacher H, Widmann S, Wessels JT, Becker V, Müller GA, and Flad T. Human □-defensins HNPs-1, -2, and -3 in renal cell carcinoma. Am J Pathol 2002, 160:1311-1324.

31. Nakamura R, Williams I, and kupper T. Keratinocyte-derived monocyte chemoattractant protein 1 (MCP-1) : analysis in a transgenic model demonstrates MCP-1 can recruit dendritic and Langerhans cells to skin. J Invest Dermatol 1995, 105:635-643.

32. Nakano T, Oka K, Arai T, Morita S, and Tsunemoto H. Prognostic significance of Langerhans' cell infiltration in radiation therapy for squamous cell carcinoma of the uterine cervix. Arch Pathol Lab Med 1989,113:507-511.

33. Nakano T, Oka K, Takahashi T, Morita S, and Arai T. Roles of Langerhans'cells and T-lymphocytes infiltrating cancer tissues in patients treated by radiation therapy for cervical cancer. Cancer 1992, 70:2839-44.

34. Oka K, Nakano T, and Arai T. Adenocarcinoma of the cervix treated with radiation alone: Prognostic significance of S-100 protein and vimentin immunostaining. Obstet Gynecol 1992, 79:347-50.

35. Parkin D, Lääza E, Muir CS. Estimates of the worlwide frequency of sixteen major cancers in 1980. Int J Cancer 1988, 41:184-197.

36.   Parody TR, Stone MJ. High level expression, activation, and antagonism of CC chemokines receptors CCR2 and CCR3 in chinese hamster ovary cells. Cytokines 2004, 27:38-46.

37.   Pater M, Pater A. Human papillomavirus types 16 and 18 sequences in carcinoma cell lines of the cervix. Virology 1985, 145:313-318.

**Claims**

1.   A mucoadhesive pharmaceutical composition comprising a polymer and a chemoattractant wherein the pH of the composition is greater than 6.

2.   A composition as defined in claim 1 wherein the chemoattractant is selected from the group consisting of MIP3$\alpha$, H$\beta$D2, MCP-1 and molgramostim.

3.   A composition as defined in claim 1 or claim 2 which contains an auxiliary chemoattractant which is selected from the group consisting of granulocyte macrophage — colony recruiting factor (GM-CSF) and HNP2 (human neutrophil defensin 2).

4.   A composition as defined in any one of the preceding claims wherein the polymer is selected from the group consisting of a natural gum, a semi-synthetic material, and a synthetic material.

5.   A composition as defined in any one of the preceding claims wherein the pH of the composition is from 6 to 8, preferably the pH of the composition is about 6.9.

6.   A composition as defined in any one of the preceding claims for use in the treatment of a squamous mucosa, preferably in the treatment of an anogenital or oral disease, especially human papillomavirus.

7.   Use of a composition as defined in any one of the preceding claims in the manufacture of a medicament for use in the treatment of a squamous mucosa, preferably in the treatment of an anogenital or oral disease, especially human papillomavirus.

8.   A method of treating an anogenital and/or oral disease which method comprises administering a therapeutically effective amount of a composition as defined in any one of claims 1 to 5 to a patient in need of such treatment.

**FIGURE 1**

**FIGURE 2**

LIQUID MEDIUM                    GEL

Figure 3

LIQUID MEDIUM                    GEL

Control

GM-CSF

MIP3α

HβD2

Figure 4

FIGURE 5

| | | | | | |
|---|---|---|---|---|---|
| HPV⁺Cells | + | + | + | + | + |
| +DC | − | + | + | + | + |
| +MCP1 | + | − | + | − | − |
| +HNP2 | + | − | − | + | − |
| +HβD2 | + | − | − | − | + |
| +MIP3α | + | − | − | − | + |

FIGURE 6

| | | | | | |
|---|---|---|---|---|---|
| PBMC | + | + | + | + | + |
| **DC** | + | + | + | + | + |
| **+MCP-1** | – | + | – | – | – |
| **+HNP2** | – | – | + | – | – |
| **+HβD2** | – | – | – | + | – |
| **+MIP3α** | – | – | – | – | + |

**European Patent Office**

## PARTIAL EUROPEAN SEARCH REPORT

which under Rule 45 of the European Patent Convention shall be considered, for the purposes of subsequent proceedings, as the European search report

Application Number

EP 04 10 6995

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 98/09642 A (THE UNITED STATES OF AMERICA, REPRESENTED BY THE S; OPPENHEIM, JOOST,) 12 March 1998 (1998-03-12)<br>* page 5, line 26 - page 8, line 20; claims 1-14 *<br>----- | 1-7 | A61K9/00 |
| A | WO 02/36141 A (IMMUNEX CORPORATION; LYNCH, DAVID H.,; DE SMEDT, THIBAUT N.,; MALISZEW) 10 May 2002 (2002-05-10)<br>* table 1 *<br>----- | 1-7 | |
| A | OH Y-K ET AL: "Enhanced mucosal and systemic immune responses to a vaginal vaccine coadministered with RANTES-expressing plasmid DNA using in situ-gelling mucoadhesive delivery system" VACCINE, BUTTERWORTH SCIENTIFIC. GUILDFORD, GB,<br>vol. 21, no. 17-18,<br>16 May 2003 (2003-05-16), pages 1989-1997, XP004421114<br>ISSN: 0264-410X<br>* the whole document *<br>----- | 1-7 | |

**TECHNICAL FIELDS SEARCHED (Int.Cl.7)**

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 3 June 2005 | Felder, C |

EPO FORM 1503 03.82 (P04C07)

**European Patent Office**

**INCOMPLETE SEARCH
SHEET C**

Application Number

EP 04 10 6995

Although claim 8 is directed to a method of treatment of the human/animal body (Article 52(4) EPC), the search has been carried out and based on the alleged effects of the compound/composition.

        - - - - -

Claim(s) searched completely:
        1-7

Claim(s) searched incompletely:
        8

Reason for the limitation of the search (non-patentable invention(s)):

Article 52 (4) EPC - Method for treatment of the human or animal body by therapy

## ANNEX TO THE EUROPEAN SEARCH REPORT
## ON EUROPEAN PATENT APPLICATION NO.

EP 04 10 6995

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2005

| Patent document cited in search report | | Publication date | Patent family member(s) | | Publication date |
|---|---|---|---|---|---|
| WO 9809642 | A | 12-03-1998 | AU<br>WO | 4251197 A<br>9809642 A2 | 26-03-1998<br>12-03-1998 |
| WO 0236141 | A | 10-05-2002 | AU<br>WO<br>US | 3244702 A<br>0236141 A2<br>2004247563 A1 | 15-05-2002<br>10-05-2002<br>09-12-2004 |

For more details about this annex : see Official Journal of the European Patent Office, No. 12/82